(19) Europäisches Patentamt
European Patent Office
Office européen des brevets

(11) **EP 4 632 748 A1**

(12) **EUROPEAN PATENT APPLICATION**

(43) Date of publication:
**15.10.2025 Bulletin 2025/42**

(21) Application number: **25156982.8**

(22) Date of filing: **10.02.2025**

(51) International Patent Classification (IPC):
***G16C 10/00*** *(2019.01)*

(52) Cooperative Patent Classification (CPC):
**G16C 10/00;** G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.04.2024 JP 2024063641**

(71) Applicant: **FUJITSU LIMITED**
**Kawasaki-shi, Kanagawa 211-8588 (JP)**

(72) Inventor: **KITAJIMA, Hironobu**
**Kawasaki-shi, 211-8588 (JP)**

(74) Representative: **Haseltine Lake Kempner LLP**
**Cheapside House**
**138 Cheapside**
**London EC2V 6BJ (GB)**

(54) **INFORMATION OUTPUT PROGRAM, INFORMATION OUTPUT METHOD, AND INFORMATION PROCESSING DEVICE**

(57) An information output program for causing a computer to execute a process includes acquiring occupancy number data that includes a time series of occupancy numbers for each of a plurality of molecular orbitals, executing principal component analysis on the occupancy number data, and outputting information on an active space that corresponds to a subset used for a quantum chemical calculation, among the plurality of molecular orbitals, based on a result of the principal component analysis.

FIG. 1

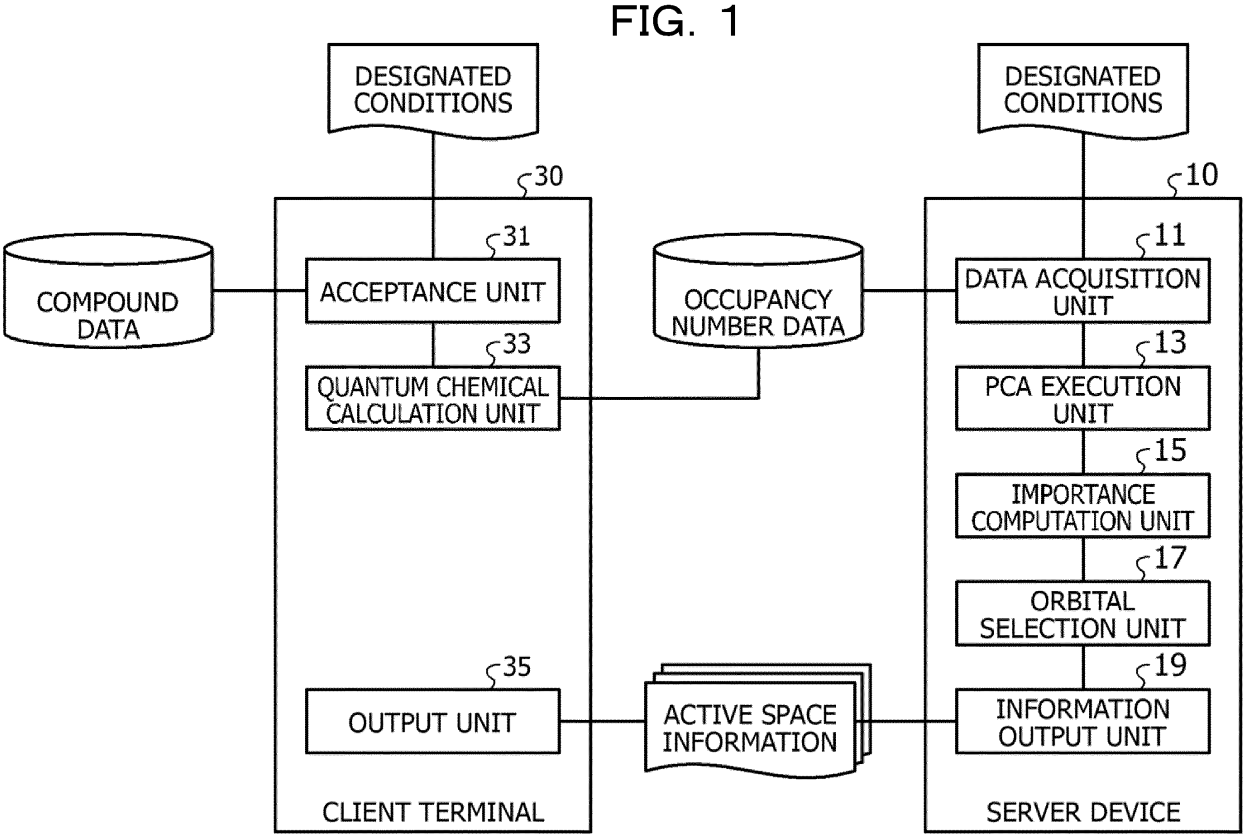


EP 4 632 748 A1

## Description

### FIELD

The embodiments discussed herein are related to an information output program, an information output method, and an information processing device.

### BACKGROUND

As one of approximation techniques for quantum chemical calculations, a molecular orbital method is known. In the molecular orbital method, an electron orbital extending over the entire molecule, which is a so-called molecular orbital, is approximately constituted by a linear bond of an electron orbital of each atom, which is a so-called atomic orbital.

For example, in the Hartree-Fock (HF) method, a wave function and orbital energy of the molecular orbital can be found using a sequential approximation technique. Electrons in an HF model are accommodated in orbitals in the order from the lowest orbital energy.

Among them, an orbital occupied by an electron and having the highest energy is called a highest occupied molecular orbital (HOMO), and an empty orbital having the lowest energy is called a lowest unoccupied molecular orbital (LUMO).

Here, in the molecular orbital method, instead of using a set of all molecular orbitals for calculation by an optimization technique such as variational calculation, variational calculation of optimization may be sometimes carried out by designating a subset of molecular orbitals, which is a so-called "active space", from the aspect of reducing the amount of calculation.

As one of such active space designation schemes, a "HOMO-m/LUMO+n type" that designates m orbitals in descending order from a HOMO and designates n orbitals in ascending order from a LUMO is known.

### Citation List

### Patent document

International Publication Pamphlet No. WO 2022/097298
Japanese Laid-open Patent Publication No. 2012-32908
U.S. Patent Application Publication No. 2020/0349459
U.S. Patent Application Publication No. 2016/0378955

### SUMMARY OF INVENTION

### TECHNICAL PROBLEM

However, since there is no definite rule or the like in the determination of the active space described above, the determination is left to the subjectivity of a user. Accordingly, the designation of the active space described above has the aspect of not necessarily being appropriate from the viewpoint of the accuracy of quantum chemical calculations, the amount of calculation, and the like.

In one aspect, it is desirable to provide an information output program, an information output method, and an information processing device capable of implementing to provide information on an active space that contributes to quantum chemical calculations.

### SOLUTION TO PROBLEM

According to an aspect of the embodiments, an information output program for causing a computer to execute a process includes acquiring occupancy number data that includes a time series of occupancy numbers for each of a plurality of molecular orbitals, executing principal component analysis on the occupancy number data, and outputting information on an active space that corresponds to a subset used for a quantum chemical calculation, among the plurality of molecular orbitals, based on a result of the principal component analysis.

ADVANTAGEOUS EFFECTS OF INVENTION

According to one embodiment, it may be possible to implement providing information on an active space that contributes to quantum chemical calculations.

BRIEF DESCRIPTION OF DRAWINGS

The invention is described, by way of example only, with reference to the following drawings, in which:

FIG. 1 is a block diagram illustrating a functional configuration example of a server device;
FIG. 2 is a schematic diagram explaining an example of a molecular orbital;
FIG. 3 is a schematic diagram explaining an example of an active space;
FIG. 4 is a schematic diagram explaining one aspect of a problem solving approach;
FIG. 5 is a flowchart illustrating a processing procedure for generating occupancy number data;
FIG. 6 is a flowchart illustrating a procedure of information output processing; and
FIG. 7 is a diagram illustrating a hardware configuration example.

DESCRIPTION OF EMBODIMENTS

Hereinafter, exemplary embodiments for carrying out an information output program, an information output method, and an information processing device according to the present disclosure will be described with reference to the accompanying drawings. Note that these exemplary embodiments merely illustrate one example or aspect, and the structures, actions, functions, properties, characteristics, methods, use purposes, and the like according to the present disclosure are not limited by such an illustrated example.

<First Exemplary Embodiment>

FIG. 1 is a block diagram illustrating a functional configuration example of a server device 10. FIG. 1 exemplifies the server device 10 that provides an information output function that implements to provide information on an active space that contributes to quantum chemical calculations.

<Explanation of Terms>

Hereinafter, prior to the description of the functional configuration example of the server device 10 illustrated in FIG. 1, some of terms related to the above-mentioned information output function in the field of quantum chemistry will be described.

(1) Molecular Orbital Method

In finding a solution to Schrodinger's equations in the field of quantum chemistry, since the interests are focused on a variety of compounds, a multibody problem is naturally involved. Therefore, there is an aspect that it is not realistic to find a strict solution, and thus a variety of ways of approximation are introduced. The molecular orbital method is one system of such approximation techniques and is one of the ideas on which current quantum chemical calculations are based.

In the molecular orbital method, an electron orbital (molecular orbital) extending over the entire molecule is approximately constituted by a linear bond of an electron orbital (atomic orbital) of each atom. In the most basic HF method, a wave function ($\varphi_i$) and orbital energy ($\varepsilon_i$) of the molecular orbital can be found using a sequential approximation technique. Note that "i" may refer to an index of the molecular orbital.

FIG. 2 is a schematic diagram explaining an example of the molecular orbital. For example, FIG. 2 illustrates, as an example, eight molecular orbitals of i = 1 to 8 corresponding to the orbital energies ($\varepsilon_1$) to ($\varepsilon_8$). As illustrated in FIG. 2, electrons are arranged in each of the eight molecular orbitals of i = 1 to 8 in the order from the lowest orbital energy. Up to two electrons can be accommodated per molecular orbital. At this time, a spin state when two electrons are placed is limited to antiparallel by the Pauli exclusion principle.

(2) Occupancy Number

**[0019]** The number of electrons arranged in each molecular orbital is called the "occupancy number". In the model of the HF method, the occupancy number takes only an integer value of zero, one, or two, but in a post-HF model such as the coupled cluster singles and doubles (CCSD) method, a real value from zero to two can be taken for one molecular orbital.

(3) HOMO and LUMO

Electrons in the HF model are accommodated in orbitals in the order from the lowest orbital energy, and one having the highest energy in orbitals occupied by electrons are called a highest occupied orbital, which is a so-called "HOMO". On the other hand, an empty orbital having the lowest energy is called a lowest empty orbital, which is a so-called LUMO.

(4) Active Space

In the molecular orbital method, an expected value of a physical quantity such as the orbital energy can be found using an optimization technique such as variational calculation. At that time, strictly, all molecular orbitals may be assumed as objects to be optimized, but the objects are often narrowed down by selecting a part of the orbitals from the aspect of the amount of calculation. A subset of the molecular orbitals thus narrowed down is called an "active space".

FIG. 3 is a schematic diagram explaining an example of the active space. In FIG. 3, as in FIG. 2, molecular orbitals corresponding to the orbital energies ($\varepsilon_1$) to ($\varepsilon_8$) are illustrated. Furthermore, in FIG. 3, molecular orbitals corresponding to the active space among eight molecular orbitals are distinguished by hatching.

As illustrated in FIG. 3, among the eight molecular orbitals of i = 1 to 8, the molecular orbitals in which electrons are arranged are the five molecular orbitals of i = 1 to 5. Among them, the molecular orbital of i = 5 having the highest orbital energy $\varepsilon_5$ is regarded as a HOMO. Meanwhile, among the three molecular orbitals of i = 6 to 8 in which no electron is arranged, the lowest orbital energy $\varepsilon_6$ is regarded as a LUMO.

For example, in the case of the example illustrated in FIG. 3, an active space is exemplified in which a range obtained by merging one molecular orbital (i = 4) in descending order from the HOMO and one molecular orbital (i = 7) in ascending order from the LUMO, that is, a subset of four molecular orbitals of i = 4 to 7, is designated.

<One Aspect of Problem>

As described in the background section above, since there is no definite rule or the like in the determination of the above-described active space, the determination is left to the subjectivity of a user. Accordingly, the designation of the active space described above has the aspect of not necessarily being appropriate from the viewpoint of the accuracy of quantum chemical calculations, the amount of calculation, and the like.

That is, the verification of estimating a range having a large influence on the accuracy of quantum chemical calculations, among the molecular orbitals disposed in descending order from the HOMO, and furthermore, a range having a large influence on the accuracy of quantum chemical calculations, among the molecular orbitals disposed in ascending order from the LUMO, is left to the subjectivity of the user.

However, it is possible at most to empirically verify an acceptable range of (m, n) due to constraints such as calculation resources and calculation time, and it is not easy even for an expert to verify which molecular orbital among molecular orbitals obtained by HF calculation or the like has a large influence on the accuracy of quantum chemical calculations.

For example, even if an expert predicts an interaction or the like between molecular orbitals by using a tool for visualizing molecular orbitals or a variety of kinds of information on molecular orbitals obtained from HF calculation or the like, it is difficult to verify the influence of individual molecular orbitals on quantum chemical calculations.

Such active space designated under the subjectivity of the user has the aspect of not necessarily being appropriate from the viewpoint of the accuracy of quantum chemical calculations, the amount of calculation, and the like.

<One Aspect of Problem Solving Approach>

Thus, the information output function according to the present exemplary embodiment acquires occupancy number data including a time series in the course of optimization of an occupancy number vector corresponding to the occupancy number of each of a plurality of molecular orbitals and outputs information on an active space, based on a result of applying principal component analysis to the occupancy number data.

Here, the above-mentioned occupancy number data may be data before convergence obtained in the course of variational optimization or the like in the molecular orbital method. This is because the variational calculation employs energy minimization or the like as an objective function and contains a characteristic change along its object even during convergence. Note that, here, the variational calculation has been taken as an example. However, since optimization may be applied to calculations other than the variational calculation, the variational calculation used for optimization, a calculation comparable to the variational calculation, and the like may be sometimes included in the category and expressed as "variational calculation or the like".

For example, an event such as changing from two or zero is unlikely to arise in the occupancy number of low energy occupied orbitals or high energy empty orbitals. Meanwhile, the closer the orbital is to the boundary with the HOMO or LUMO, the closer the value of energy is. Accordingly, a change in the occupancy number arises at a high frequency, and thus, variations over time occur.

By applying, to such occupancy number data, principal component analysis for dimensionally reducing high-dimensional data to low-dimensional data by extracting a characteristic that most exactly represents variations in the whole, it is enabled to distinguish between molecular orbitals having a large influence on the accuracy of quantum chemical calculations and molecular orbitals having no such influence.

FIG. 4 is a schematic diagram explaining one aspect of a problem solving approach. FIG. 4 illustrates a graph in which data points corresponding to an occupancy number vector including occupancy numbers $x_1$ to $x_3$ of three molecular orbitals of $i = 1$ to 3 as elements are plotted with black circle marks for each iteration of the variational calculation or the like. Furthermore, FIG. 4 illustrates data axes $t_1$ to $t_3$ corresponding to a first principal component to a third principal component obtained as a result of applying the principal component analysis.

As illustrated in FIG. 4, the data group of the occupancy number vector is concentrated and distributed on a two-dimensional plane formed by two data axes $t_1$ and $t_2$. From this, it is clear that what variations the data group of the occupancy number vector in three dimensions of $X_1$ to $X_3$ has can be favorably represented only by the two axes of $(t_1, t_2)$.

Here, FIG. 4 takes an example in which the number of dimensions, that is, the number of molecular orbitals is "3" for convenience of description, but the occupancy number vector that can be collected from the course of actual variational optimization or the like can have a higher-dimensional space than such a three-dimensional space.

However, even if the number of dimensions of the occupancy number vector increases, the trend that the occupancy numbers of low energy occupied orbitals or high energy empty orbitals hardly change is not lost, so that it is clear that similar dimensional concentration of variations occurs.

Since the molecular orbital in which a change in the occupancy number occurs at a high frequency can be specified based on a data axis where the variations are concentrated in this manner, it is possible to output information regarding a molecular orbital having a large influence on the accuracy of quantum chemical calculations and a molecular orbital having no such influence.

As one aspect, it is obvious that the calculation accuracy is enhanced by selecting, as the active space, a molecular orbital having a large influence on the accuracy of quantum chemical calculations among all molecular orbitals. Furthermore, even if the number of molecular orbitals designated as the active space is reduced due to constraints such as calculation resources and calculation time, it is also obvious that deterioration of the calculation accuracy may be suppressed by selecting a molecular orbital having a large influence on the accuracy of quantum chemical calculations, as the active space.

Therefore, according to the information output function according to the present exemplary embodiment, it may be possible to implement providing information on an active space that contributes to quantum chemical calculations in diverse aspects such as the calculation accuracy, calculation resources, and calculation time. By providing such information on the active space, the accuracy and the time taken for quantum chemical calculations in the molecular orbital method may be balanced even without a high degree of specialized knowledge regarding quantum chemical calculations, a skill regarding software of quantum chemical calculations, or the like. Consequently, automatic designation of the active space may be enabled while mitigating dependence on individual expertise and experience.

<Overall Configuration>

Merely as an example of use cases, FIG. 1 illustrates a case where the server device 10 provides the above-described information output function, based on the occupancy number data obtained in the course of the variational calculation or the like of quantum chemical calculations executed by a client terminal 30.

The server device 10 is an example of an information processing device that provides the above-described information output function. For example, the server device 10 can be implemented as a software as a service (SaaS) type application. This may allow the above-described information output function to be provided as a cloud service. Besides, the server device 10 is not excluded from providing the above-described information output function on-premises.

The client terminal 30 is an example of a computer that are provided with the above-described information output function. A user of such an information output function may be any person concerned in carrying out quantum chemical calculations in the molecular orbital method. For example, an employee of a manufacturer of a chemical product, a pharmaceutical, or the like, such as an expert like a developer may be included.

<Configuration of Client Terminal 30>

Next, a functional configuration example of the client terminal 30 according to the present exemplary embodiment will be described. FIG. 1 schematically depicts blocks related to a function related to a function of generating the occupancy number data included in the client terminal 30.

As illustrated in FIG. 1, the client terminal 30 includes an acceptance unit 31, a quantum chemical calculation unit 33, and an output unit 35. Note that FIG. 1 merely illustrates excerpted functional units related to functions corresponding to the above-described function of generating the occupancy number data, and functional units other than those illustrated may be included in the client terminal 30.

The acceptance unit 31 is a processing unit that accepts various types of requests. For example, the acceptance unit 31 can accept a request demanding the execution of quantum chemical calculations, via a user interface (not illustrated).

At the time of acceptance of such a request, the acceptance unit 31 can accept an input of "compound data" representing a three-dimensional structure of a molecule treated as a target for quantum chemical calculations. For example, the compound data may include types of atoms constituting the compound, XYZ coordinates of atoms, and the like. Furthermore, an input of parameters used at the time of execution of quantum chemical calculations, such as "designated conditions" including the number of iterations and the active space, for example, may be accepted.

The quantum chemical calculation unit 33 is a processing unit that executes quantum chemical calculations. As an embodiment, the quantum chemical calculation unit 33 can generate the occupancy number data by executing software that implements quantum chemical calculations in accordance with the above compound data and the above designated conditions. Such quantum chemical calculation software may be any existing software regardless of being open source or from a particular vendor.

Here, the quantum chemical calculation executed by the quantum chemical calculation unit 33 may be distinguished from the quantum chemical calculations executed in accordance with the designation of an active space after the determination of the active space, for the reasons mentioned below, and algorithms of the two types of quantum chemical calculations and parameters used in the two types of quantum chemical calculations may be different.

As one aspect, the quantum chemical calculation executed by the quantum chemical calculation unit 33 has an aspect that the quantum chemical calculation does not have to be iterated until optimization of the variational calculation or the like in the molecular orbital method converges. From such an aspect, as one of the above-mentioned designated conditions, any number of iterations equal to or more than one iteration can be designated as the number of iterations of the variational calculation or the like.

As another aspect, the quantum chemical calculation executed by the quantum chemical calculation unit 33 is sufficient with calculation accuracy to an extent that allows the orbital energy to be calculated. From such an aspect, a higher-speed algorithm than the quantum chemical calculations executed after the determination of the active space may be designated as one of the above-mentioned designated conditions. For example, the active space applied to the Variational Quantum Eigensolver (VQE) method can also be acquired from the course of calculation of the post-HF model such as the higher-speed CCSD method.

As a further aspect, in a use scene with a large amount of calculation with all molecular orbitals as a balance with respect to calculation resources due to a large scale of a compound or other factors, a case where even one iteration of the variational calculation or the like is not allowed to be executed can also be supposed. In this case, as one of the above-mentioned designated conditions, the designation of the active space can be accepted in the HOMO-m/LUMO+n type. Besides, it is also possible to acquire an execution result for one iteration of the variational calculation or the like by accepting designation of region division such as density matrix embedding theory (DMET).

The output unit 35 is a functional unit that outputs various types of information. As one aspect, the output unit 35 can execute display output, sound output, or print output of active space information output by an information output unit 19 of the server device 10.

Note that, here, an example in which the active space information is presented to the user has been taken as one aspect of information output, but it goes without saying that the active space information can be output to software or a service that executes quantum chemical calculations in accordance with the designation of the active space. In this case, the quantum chemical calculations can be executed by skipping confirmation and editing of the active space by the user.

<Configuration of Server Device 10>

Next, a functional configuration example of the server device 10 according to the present exemplary embodiment will be described. FIG. 1 schematically depicts blocks related to a function related to the information output function that the server device 10 has.

As illustrated in FIG. 1, the server device 10 includes a data acquisition unit 11, a principal component analysis (PCA) execution unit 13, an importance computation unit 15, an orbital selection unit 17, and the information output unit 19. Note that FIG. 1 merely illustrates excerpted functional units related to functions corresponding to the above-described information output function, and functional units other than those illustrated may be included in the server device 10.

The data acquisition unit 11 is a processing unit that acquires the occupancy number data described above. As an embodiment, the data acquisition unit 11 can acquire the above occupancy number data among the execution results of the quantum chemical calculation by the quantum chemical calculation unit 33 of the client terminal 30. Such acquisition of the occupancy number data may be executed on demand, or may be automatically executed in cooperation with the quantum chemical calculation unit 33 of the client terminal 30.

The PCA execution unit 13 is a processing unit that executes principal component analysis, which is so-called PCA. Such PCA may be implemented by any variation of models, including linear models, and may be executed by any software for multivariate analysis. As one embodiment, the PCA execution unit 13 executes the PCA on the occupancy number data acquired by data acquisition unit 11, as will be described below.

That is, it is considered to compress an attribution dimension of a data matrix $X \in R^{n \times p}$ constituted by data of n points having p attributes to q from p ($q \leq p$) in line with the idea of principal component analysis (PCA). Note that, here, it is supposed that an occupancy number vector including the occupancy numbers of p orbitals is used as data points $x_i = (x_{i1}, x_{i2}, ..., x_{ip})$ with i = 1, 2, ..., n.

In the principal component analysis, an object is to extract a component that exactly exhibits a characteristic of the raw data from a structure that the raw data has, by some linear transformation. Assuming that a data matrix after the transformation is $T \in R^{n \times p}$ and a factor loading matrix denoting the linear transformation for compression is $W \in R^{p \times p}$, there is a relationship of T = XW between these data matrix and factor loading matrix. While the vectors $t_i = (t_{i1}, t_{i2}, ..., t_{ip})$ with i = 1, 2, ..., n after data transformation are called principal component scores, and T is called principal component score matrix, following Formulas (1) to (3) are obtained when each matrix is explicitly indicated.

[Mathematical Formula 1]

$$T = \begin{pmatrix} t_{11} & t_{12} & \cdots & t_{1p} \\ t_{21} & t_{22} & \cdots & t_{2p} \\ \vdots & \vdots & \ddots & \vdots \\ t_{n1} & t_{n2} & \cdots & t_{np} \end{pmatrix}, X = \begin{pmatrix} x_{11} & x_{12} & \cdots & x_{1p} \\ x_{21} & x_{22} & \cdots & x_{2p} \\ \vdots & \vdots & \ddots & \vdots \\ x_{n1} & x_{n2} & \cdots & x_{np} \end{pmatrix}, W = \begin{pmatrix} w_{11} & w_{12} & \cdots & w_{1p} \\ w_{21} & w_{22} & \cdots & w_{2p} \\ \vdots & \vdots & \ddots & \vdots \\ w_{p1} & w_{p2} & \cdots & w_{pp} \end{pmatrix} \quad \cdots (1) \sim (3)$$

At the time of the dimension reduction described above, it is ensured that information on variations in fluctuations of the data points $x_i = (x_{i1}, x_{i2}, ..., x_{ip})$ with i = 1, 2, ..., n be not lost as much as possible. Specifically, linear transformation W intended to make sample variance of the principal component score vectors $t_i$ have as large a value as possible is found and applied. This correlates to defining a q-dimensional space regarded to most exactly save how the original data points are distributed in the p-dimensional space.

According to the theory of the principal component analysis (PCA), such a transformation matrix Wq for compression of dimensions can be obtained by solving a variance-covariance matrix S with p attribute variables, that is, an eigenvalue problem of following Formula (4).

[Mathematical Formula 2]

$$S = \begin{pmatrix} \sigma_{11} & \sigma_{12} & \cdots & \sigma_{1p} \\ \sigma_{21} & \sigma_{22} & \cdots & \sigma_{2p} \\ \vdots & \vdots & \ddots & \vdots \\ \sigma_{p1} & \sigma_{p2} & \cdots & \sigma_{pp} \end{pmatrix} \quad \cdots (4)$$

Here, $\sigma_{ij}$ = (ij = 1, 2, ..., p) denotes a value of covariance when the i-th and j-th attribute variables are deemed as random variables. When assuming that an eigenvalue of S that is a square matrix is $\lambda$ and an eigenvector thereof is w (which may be normalized to $|w| = 1$), a solution of the equation $Sw = \lambda w$ can be easily found using an existing technology.

Assuming that $\lambda_1, \lambda_2, ..., \lambda_p$ ($\lambda_1 \geq \lambda_2, ..., \geq \lambda_p$) and $w_1, w_2, ..., w_p$ are obtained by disposing eigenvalues and eigenvectors corresponding to these eigenvalues in order of magnitude of the eigenvalues, the dimensions of the raw data can be compressed by selecting q ($\leq$ p) eigenvalues and eigenvectors in descending order from the maximum eigenvalue. The transformation matrix of the data transformation formula: $T_q = XW_q$ can be determined by disposing column vectors as in $W_q = (w_1, w_2, ..., w_q)$ with $w_i \in R^p$, while the principal component score matrix T is also pruned to $T_q = (t_1, t_2, ..., t_q)$ with $t_i \in R^n$ at the same time.

Here, the principal component score $t_1$ corresponding to the maximum eigenvalue (first eigenvalue) is called a first principal component and is assumed to most exactly denote how the raw data fluctuates. Hereinafter, similarly, the vector $t_j$ is called a j-th principal component, where it is considered that the influence of the vector $t_j$ is weakened because

the eigenvalue becomes smaller as j increases. In addition, it is known that the j-th eigenvalue found here coincides with the variance of the j-th principal component, and the axes of the respective principal components are all orthogonal.

If a vector containing the occupancy numbers of p orbitals are given as the above data points $x_i = (x_{i1}, x_{i2}, ..., x_{ip})$ with i = 1, 2, ..., n, the dimensions of the data points can be compressed to q from p. Here, its first principal component $t_1 = XW_1$ ($t_1 \in R^n$, $X \in R^{n \times p}$, $W_1 \in R^{p \times 1}$) is represented by a matrix as following Formula (5). The first principal component denotes a component of each data point when a direction in which the variance of the raw data is maximized is assumed as a coordinate axis.

[Mathematical Formula 3]

$$t_1 = \begin{pmatrix} t_{11} \\ t_{21} \\ \vdots \\ t_{n1} \end{pmatrix} = \begin{pmatrix} x_{11} & x_{12} & \cdots & x_{1p} \\ x_{21} & x_{22} & \cdots & x_{2p} \\ \vdots & \vdots & \ddots & \vdots \\ x_{n1} & x_{n2} & \cdots & x_{np} \end{pmatrix} \begin{pmatrix} w_{11} \\ w_{21} \\ \vdots \\ w_{p1} \end{pmatrix} \quad \cdots (5)$$

The importance computation unit 15 is a processing unit that computes the importance of the molecular orbital, based on the eigenvalues and the values of the eigenvectors of a predetermined number of principal components from the first principal component obtained as a result of the principal component analysis by the PCA execution unit 13.

The first principal component correlates to a component that most dominates fluctuations in the original data, and the influence of the occupancy numbers of p orbitals of the raw data on this component is quantified. Since the occupancy number of the i-th orbital is multiplied by the coefficients $w_{j1}$ with j = 1, 2, ..., p in above Formula (5), quantification is possible by some function of these coefficients. In addition, from the reasons that the positive and negative do not have to be considered for fluctuations in the occupancy number, and variance of the first principal component has a linear sum with $w_{i1}^2$ of the variance of the occupancy number as a count (it is tentatively assumed that fluctuations of each orbital occupancy number are independent), it can be seen that $w_{j1}^2$ is suitable as a criterion for quantification. Such a coefficient $w_{j1}^2$ can be computed as the importance of the molecular orbital.

The computation of the importance based only on the first principal component has been exemplified thus far, but the importance based on a plurality of principal components can also be computed. For example, in a case where comprehensive evaluation including the first principal component to a K-th principal component is implemented, an importance index v(j) of the orbital j can be computed in accordance with the computation formula of Formula (6) below. Since $\lambda_k$ of the k-th eigenvalue coincides with the variance of the k-th principal component score, this can correspond to a proportional distribution of the value of $\lambda_k$ by the magnitude of $w_{j1}^2$, which is also used in the computation scheme according to only the first principal component.

[Mathematical Formula 4]

$$v(j) = \sum_{k=1}^{K} \frac{\lambda_k w_{jk}^2}{|w_k|^2}, |w_k|^2 = \sum_{j=1}^{p} w_{jk}^2, j = 1,2,\cdots,p \quad \cdots (6)$$

Note that, by normalizing as $|w_k|^2 = 1, 2, ..., K$, this index can also be simplified as indicated by following Formula (7). When the active space is actually determined, this v(j) only has to be computed individually for each orbital j. This v(j) correlates to taking, for the j-th attribute variable, the sum of squares of the factor loading of the first to K-th principal components referred to in the principal component analysis.

[Mathematical Formula 5]

$$v(j) = \sum_{k=1}^{K} \lambda_k w_{jk}^2 \quad \cdots (7)$$

The orbital selection unit 17 is a processing unit that selects molecular orbitals of which importance computed by the importance computation unit 15 falls within a predetermined number of top ranks, among a plurality of molecular orbitals. As one embodiment, the orbital selection unit 17 can select a number of orbitals j equal to the predetermined number of top ranks, in the order from one having the highest importance v(j) of the molecular orbital computed by the importance computation unit 15. This may implement automatic determination of the active space.

The information output unit 19 is a processing unit that executes information output to the client terminal 30. Merely as an example, the information output unit 19 can output, to the client terminal 30, a list of the indices j of the molecular orbitals selected by the orbital selection unit 17, a list of the importance v(j) of each molecular orbital j, the variance of the occupancy number of each molecular orbital j, or a combination thereof, as active space information. Note that, here, an example of outputting the active space information regarding the molecular orbitals selected by the orbital selection unit 17 has been mentioned, but by outputting the active space information on all the orbitals j, the user is also allowed to edit the active space used for quantum chemical calculations.

<Flow of Processing>

Next, a flow of processing executed by each device according to the present exemplary embodiment will be described. Here, (1) processing for generating the occupancy number data executed by the client terminal 30 will be described, and then, (2) information output processing executed by the server device 10 will be described.

(1) Processing for Generating Occupancy Number Data

FIG. 5 is a flowchart illustrating processing for generating the occupancy number data. This processing can be started, merely as an example, in a case where a request demanding the execution of quantum chemical calculations is accepted by the acceptance unit 31.

As illustrated in FIG. 5, the quantum chemical calculation unit 33 reads compound data input at the time of accepting the above request and also reads the designated conditions designating the number of iterations of the variational calculation or the like, the active space, and the like (steps S101 and S102). Subsequently, the quantum chemical calculation unit 33 sets an initial state of the quantum chemical calculation (step S103).

Thereafter, the quantum chemical calculation unit 33 executes loop processing 1 of iterating following step S104 by the number of times corresponding to the number of iterations N included in the designated conditions read in step S102. That is, the quantum chemical calculation unit 33 executes n-th variational optimization and the like (step S104).

By iterating such loop processing 1, a data matrix constituted by a time series of n points of the occupancy number vector including the occupancy numbers of p molecular orbitals as elements is obtained as the occupancy number data.

Thereafter, the quantum chemical calculation unit 33 outputs the occupancy number data obtained in the loop processing 1 to the server device 10 (step S105) and ends the processing.

(2) Information Output Processing

FIG. 6 is a flowchart illustrating a procedure of the information output processing. This processing can be started, merely as an example, in a case where the occupancy number data described above is acquired from the client terminal 30.

As illustrated in FIG. 6, the PCA execution unit 13 reads the data matrix X of the occupancy numbers of all orbitals acquired by the data acquisition unit 11 (step S301). Subsequently, the PCA execution unit 13 calculates the variance-covariance matrix S of the occupancy numbers from the data matrix X (step S302).

Then, the PCA execution unit 13 executes numerical calculation of an algorithm that solves the eigenvalue problem regarding the variance-covariance matrix S (step S303). Thereafter, the PCA execution unit 13 sorts the eigenvalues $\lambda_i$ obtained as a result of the calculation in step S303 and the corresponding eigenvectors $w_i$ in descending order of the magnitude of $\lambda_i$ (step S304).

Subsequently, the importance computation unit 15 calculates the importance index v(j) of each orbital j from the eigenvalues and the values of the eigenvectors from the first principal component to the K-th principal component (step S305). After that, the orbital selection unit 17 selects a number of orbitals j equal to a predetermined number L in the order from one having the largest importance index v(j) (step S306).

Thereafter, the information output unit 19 outputs active space information including a number of indices of the orbitals j equal to L, a list of the importance v(j) of the respective orbitals j, and the like, to the client terminal 30 (step S307), and ends the processing.

<One Aspect of Effects>

As described above, the server device 10 according to the present exemplary embodiment acquires the occupancy number data including a time series of the occupancy number vector corresponding to the occupancy number of each of a plurality of molecular orbitals and outputs information on an active space, based on a result of applying the principal component analysis to the occupancy number data.

Therefore, according to the server device 10 according to the present exemplary embodiment, it may be possible to implement providing information on an active space that contributes to quantum chemical calculations in diverse aspects such as the calculation accuracy, calculation resources, and calculation time. By providing such information on the active space, the accuracy and the time taken for quantum chemical calculations in the molecular orbital method may be balanced even without a high degree of specialized knowledge regarding quantum chemical calculations, a skill regarding software of quantum chemical calculations, or the like. Consequently, automatic designation of the active space may be enabled while mitigating dependence on individual expertise and experience.

<Second Exemplary Embodiment>

Incidentally, while the first exemplary embodiment of the present disclosure has been described thus far, various applications are possible, and furthermore, the first exemplary embodiment may also be carried out in various different forms other than the first exemplary embodiment described above.

<Demonstration of Creativity>

The matters described in the above first exemplary embodiment, such as specific examples or the like of the types and parameters of the quantum chemical calculations or the principal component analysis algorithm, for example, are merely examples and can be altered. In addition, also in the flowcharts described in the first exemplary embodiment, the order of processing can be altered within a range without contradiction.

<System>

Pieces of information including the processing procedures, control procedures, specific names, various types of data and parameters described above or illustrated in the drawings can be altered in any way unless otherwise noted. For example, any one or more functional units among the data acquisition unit 11, the PCA execution unit 13, the importance computation unit 15, the orbital selection unit 17, and the information output unit 19 included in the server device 10 may be configured as separate devices.

In addition, each constituent element of each device illustrated in the drawings is functionally conceptual and does not necessarily have to be physically configured as illustrated in the drawings. That is, specific forms of distribution and integration of respective devices are not restricted to the forms illustrated in the drawings. In other words, all or some of the devices can be configured by being functionally or physically distributed or integrated in any units according to various types of loads, use situations, or the like. Note that each configuration may be a physical configuration.

For example, in the first exemplary embodiment described above, an example has been mentioned in which the above-described generation of the occupancy number data is executed by the client terminal 30, but the server device 10 can also execute the above generation of the occupancy number data collectively. Furthermore, in the first exemplary embodiment described above, an example has been mentioned in which the server device 10 outputs the active space information to the client terminal 30, but the server device 10 may execute quantum chemical calculations, based on the active space information.

Moreover, all or any part of individual processing functions performed by each device can be implemented by a central processing unit (CPU) and a program analyzed and executed by the CPU, or can be implemented as hardware by wired logic.

<Hardware>

Next, a hardware configuration example of the computer mentioned in the first and second exemplary embodiments will be described. FIG. 7 is a diagram illustrating a hardware configuration example. As illustrated in FIG. 7, the server device 10 includes a communication device 10a, a storage device 10b, a memory 10c, and a processor 10d. Note that the respective units illustrated in FIG. 7 may be mutually coupled by a bus or the like.

The communication device 10a is a network interface card or the like. The storage device 10b is a storage device such as a hard disk drive (HDD) or a solid state drive (SSD). For example, the storage device 10b stores a program for operating the functions illustrated in FIG. 1, a database (DB), and the like.

The processor 10d reads a program that executes processing similar to the processing of the processing units illustrated in FIG. 1, from the storage device 10b or the like, and loads the read program into the memory 10c, thereby operating a process that executes the functions described with reference to FIG. 1.

Such a process implements functions similar to the functions of the processing units included in the server device 10. For example, the processor 10d reads a program having functions similar to the functions of the data acquisition unit 11, the PCA execution unit 13, the importance computation unit 15, the orbital selection unit 17, the information output unit 19, and the like, from the storage device 10b or the like. Then, the processor 10d executes a process of executing processing similar to the processing of the data acquisition unit 11, the PCA execution unit 13, the importance computation unit 15, the orbital selection unit 17, the information output unit 19, and the like.

As described above, the server device 10 operates as an information processing device that executes an information output method by reading and executing the program. In addition, the server device 10 can also implement functions similar to the functions of the first exemplary embodiment described above, by reading the above-mentioned program from a recording medium with a medium reading device and executing the above-read program. Note that the program referred to in the second exemplary embodiment is not limited to being executed by the server device 10. For example, the functions of the present disclosure can be similarly applied also to a case where another computer or server executes the program, or a case where such computer and server cooperatively execute the program.

The program described above may be distributed via a network such as the Internet. In addition, the program described above can be executed by being recorded on any recording medium and read from the recording medium by the computer. For example, the recording medium can be implemented by a hard disk, a flexible disk (FD), a compact disc read only memory (CD-ROM), a magneto-optical disk (MO), a digital versatile disc (DVD), or the like.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

## Claims

**1.** An information output program for causing a computer to execute a process comprising:

acquiring occupancy number data that includes a time series of occupancy numbers for each of a plurality of molecular orbitals;
executing principal component analysis on the occupancy number data; and
outputting information on an active space that corresponds to a subset used for a quantum chemical calculation, among the plurality of molecular orbitals, based on a result of the principal component analysis.

**2.** The information output program according to claim 1, for causing the computer to further execute the process comprising computing importance of the molecular orbitals, based on eigenvalues and values of eigenvectors of a predetermined number of principal components from a first principal component obtained as the result of the principal component analysis.

**3.** The information output program according to claim 2, for causing the computer to further execute the process comprising selecting the molecular orbitals of which the importance falls within a predetermined number of top ranks, among the plurality of molecular orbitals, wherein
the outputting includes outputting information regarding the molecular orbitals selected in the selecting.

**4.** The information output program according to any of the preceding claims, wherein the outputting includes outputting the information regarding indices of the molecular orbitals, the importance of the molecular orbitals or a variance of the occupancy numbers of the molecular orbitals or any combination of the indices of the molecular orbitals, the importance of the molecular orbitals or the variance of the occupancy numbers of the molecular orbitals.

**5.** The information output program according to any of the preceding claims, wherein the occupancy number data corresponds to an execution result obtained by iterative calculation that includes variational optimization in a molecular orbital method.

**6.** The information output program according to claim 5, wherein the occupancy number data is data before convergence obtained in a course of the iterative calculation.

**7.** An information output method executed by a computer, the information output method comprising:

acquiring occupancy number data that includes a time series of occupancy numbers for each of a plurality of molecular orbitals;
executing principal component analysis on the occupancy number data; and
outputting information on an active space that corresponds to a subset used for a quantum chemical calculation, among the plurality of molecular orbitals, based on a result of the principal component analysis.

**8.** The information output method according to claim 7, further comprising computing importance of the molecular orbitals, based on eigenvalues and values of eigenvectors of a predetermined number of principal components from a first principal component obtained as the result of the principal component analysis.

**9.** The information output method according to claim 8, further comprising selecting the molecular orbitals of which the importance falls within a predetermined number of top ranks, among the plurality of molecular orbitals, wherein the outputting includes outputting information regarding the molecular orbitals selected in the selecting.

**10.** A information processing device comprising:

a processing unit to
acquire occupancy number data that includes a time series of occupancy numbers for each of a plurality of molecular orbital,
execute principal component analysis on the occupancy number data, and
output information on an active space that corresponds to a subset used for a quantum chemical calculation, among the plurality of molecular orbitals, based on a result of the principal component analysis.

**11.** The information processing device according to claim 10, the processing unit further configured to computing importance of the molecular orbitals, based on eigenvalues and values of eigenvectors of a predetermined number of principal components from a first principal component obtained as the result of the principal component analysis.

**12.** The information processing device according to claim 11, the processing unit further configured to selecting the molecular orbitals of which the importance falls within a predetermined number of top ranks, among the plurality of molecular orbitals, wherein
the processing unit outputs information regarding the molecular orbitals selected in the selecting.

**13.** The information processing device according to any of claims 10 to 12, wherein the processing unit output the information regarding indices of the molecular orbitals, the importance of the molecular orbitals or a variance of the occupancy numbers of the molecular orbitals or any combination of the indices of the molecular orbitals, the importance of the molecular orbitals or the variance of the occupancy numbers of the molecular orbitals.

**14.** The information processing device according to any of claims 10 to 13, wherein the occupancy number data corresponds to an execution result obtained by iterative calculation that includes variational optimization in a molecular orbital method.

**15.** The information processing device according to claim 14, wherein the occupancy number data is data before convergence obtained in a course of the iterative calculation.

FIG. 1

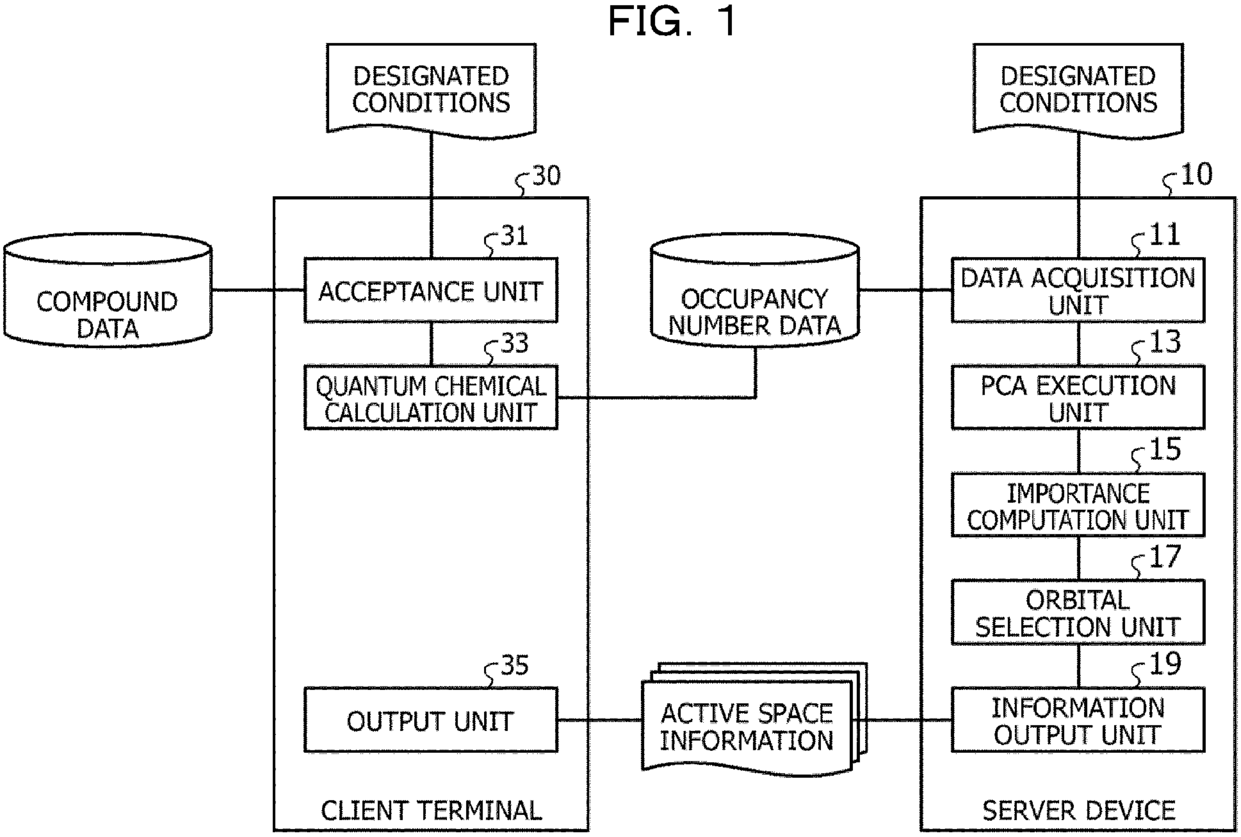
DESIGNATED CONDITIONS
30
31
COMPOUND DATA
ACCEPTANCE UNIT
33
QUANTUM CHEMICAL CALCULATION UNIT
35
OUTPUT UNIT
CLIENT TERMINAL
OCCUPANCY NUMBER DATA
ACTIVE SPACE INFORMATION
DESIGNATED CONDITIONS
10
11
DATA ACQUISITION UNIT
13
PCA EXECUTION UNIT
15
IMPORTANCE COMPUTATION UNIT
17
ORBITAL SELECTION UNIT
19
INFORMATION OUTPUT UNIT
SERVER DEVICE

FIG. 2

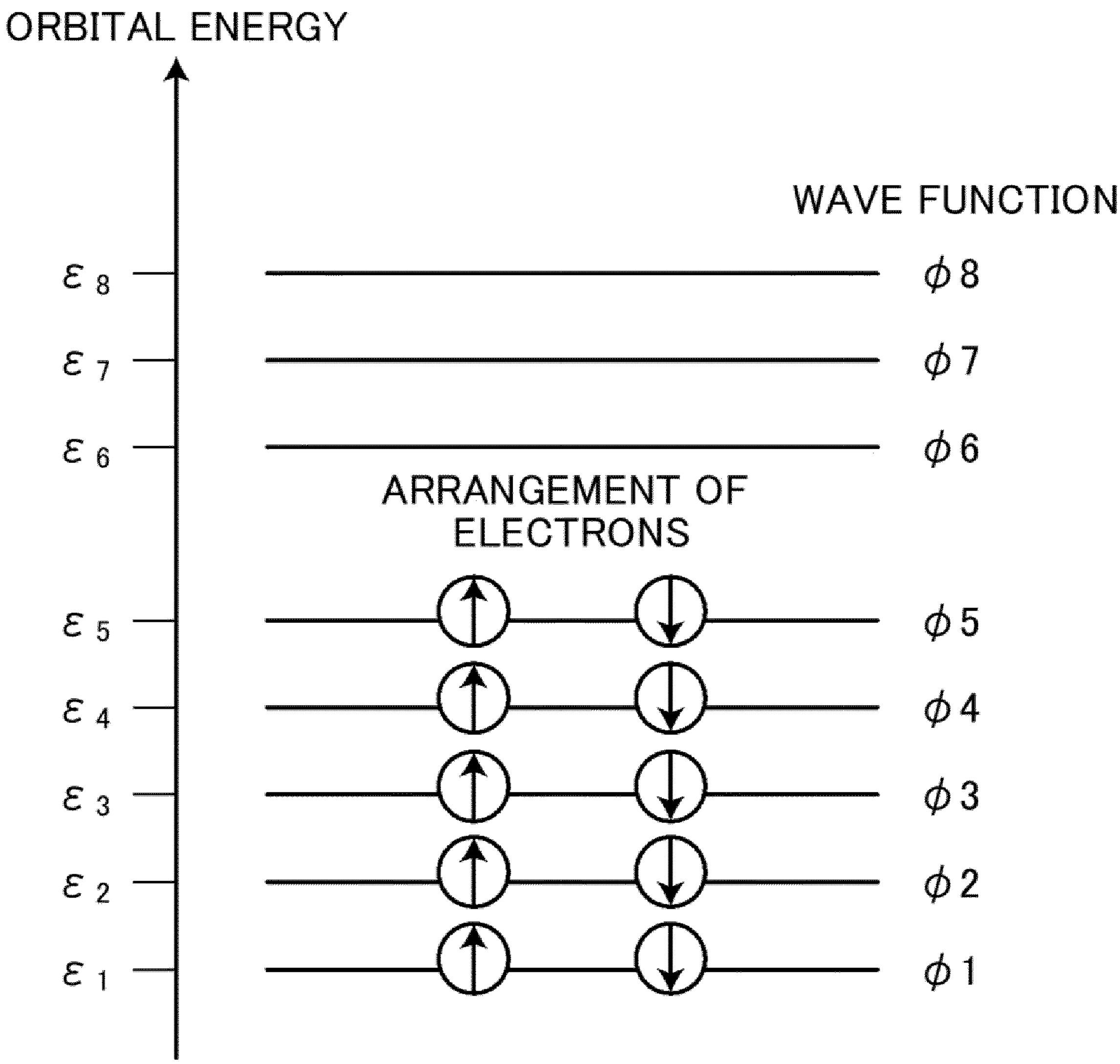
ORBITAL ENERGY
WAVE FUNCTION
ε 8
ε 7
ε 6
ε 5
ε 4
ε 3
ε 2
ε 1
φ8
φ7
φ6
φ5
φ4
φ3
φ2
φ1
ARRANGEMENT OF ELECTRONS

FIG. 3

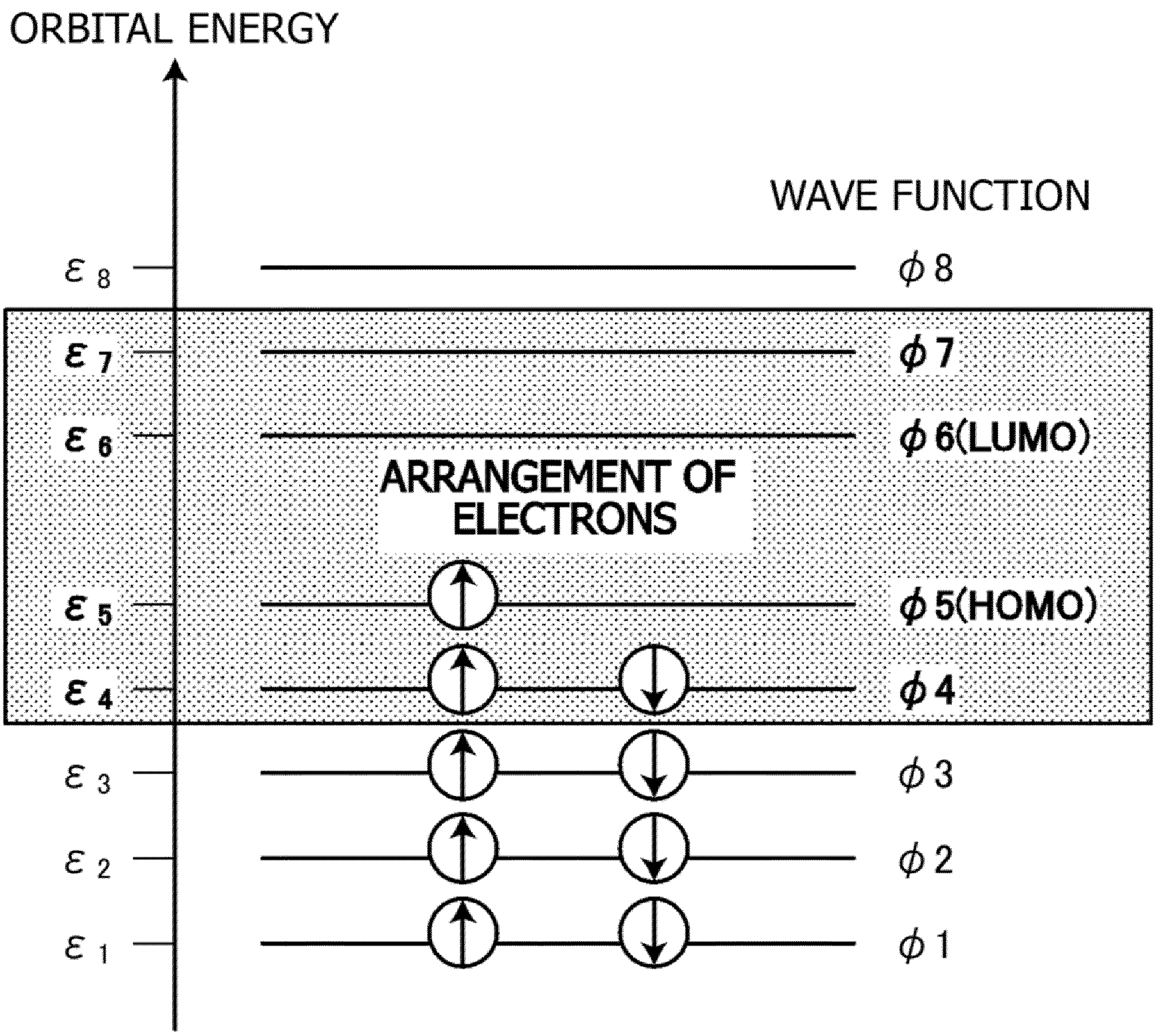
ORBITAL ENERGY
WAVE FUNCTION
ε 8
ε 7
ε 6
ε 5
ε 4
ε 3
ε 2
ε 1
ARRANGEMENT OF ELECTRONS
φ 8
φ 7
φ 6(LUMO)
φ 5(HOMO)
φ 4
φ 3
φ 2
φ 1

FIG. 4

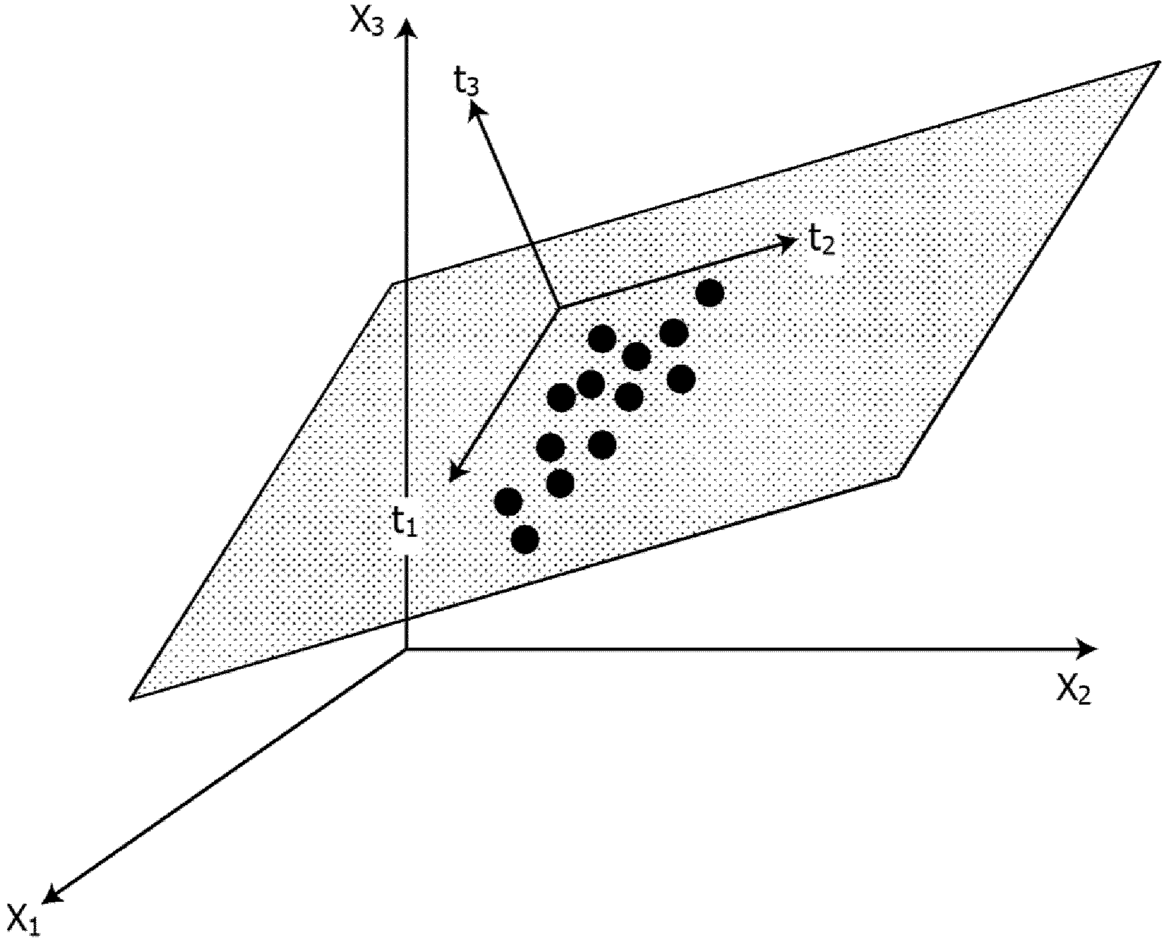

FIG. 5

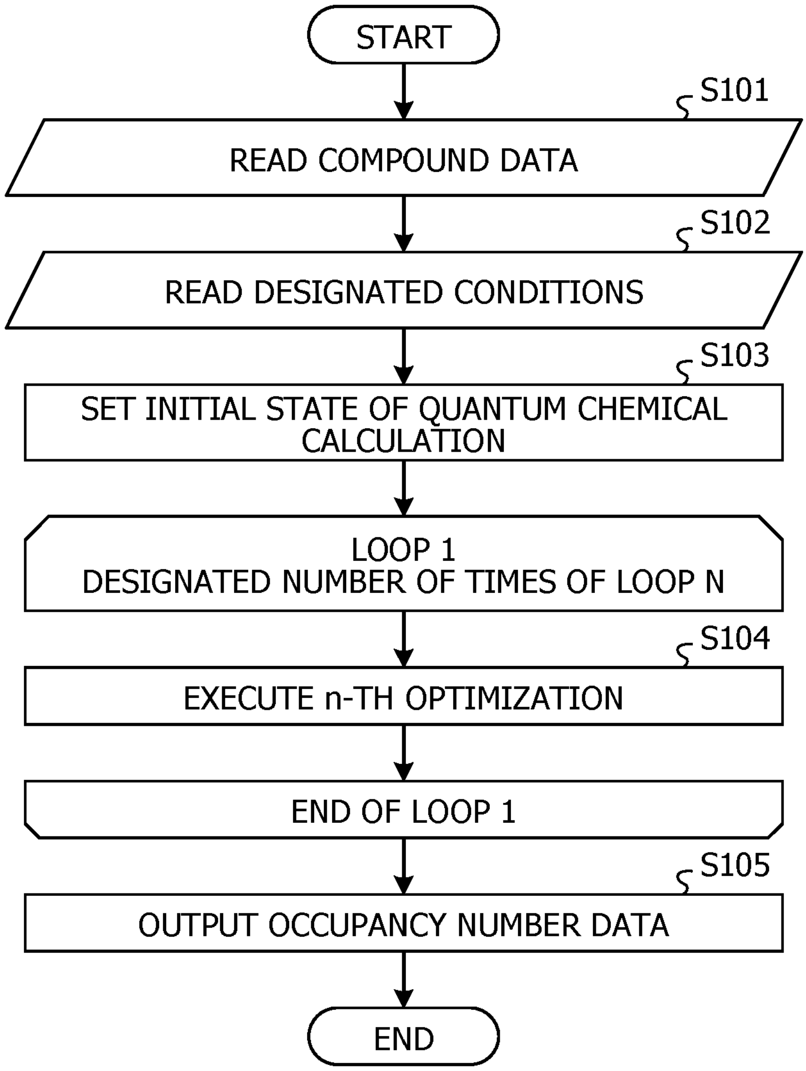
START
S101
READ COMPOUND DATA
S102
READ DESIGNATED CONDITIONS
S103
SET INITIAL STATE OF QUANTUM CHEMICAL CALCULATION
LOOP 1
DESIGNATED NUMBER OF TIMES OF LOOP N
S104
EXECUTE n-TH OPTIMIZATION
END OF LOOP 1
S105
OUTPUT OCCUPANCY NUMBER DATA
END

FIG. 6

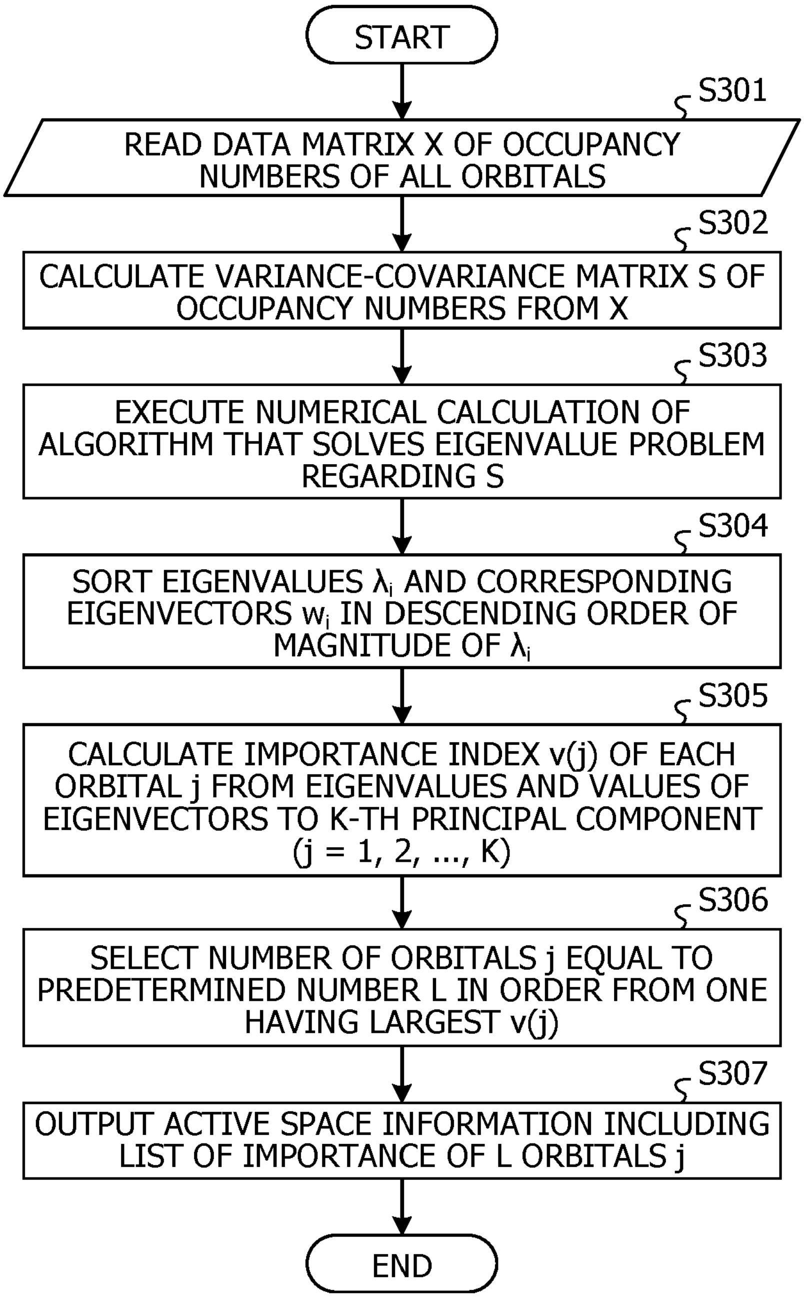
START
S301
READ DATA MATRIX X OF OCCUPANCY NUMBERS OF ALL ORBITALS
S302
CALCULATE VARIANCE-COVARIANCE MATRIX S OF OCCUPANCY NUMBERS FROM X
S303
EXECUTE NUMERICAL CALCULATION OF ALGORITHM THAT SOLVES EIGENVALUE PROBLEM REGARDING S
S304
SORT EIGENVALUES $\lambda_i$ AND CORRESPONDING EIGENVECTORS $w_i$ IN DESCENDING ORDER OF MAGNITUDE OF $\lambda_i$
S305
CALCULATE IMPORTANCE INDEX v(j) OF EACH ORBITAL j FROM EIGENVALUES AND VALUES OF EIGENVECTORS TO K-TH PRINCIPAL COMPONENT (j = 1, 2, ..., K)
S306
SELECT NUMBER OF ORBITALS j EQUAL TO PREDETERMINED NUMBER L IN ORDER FROM ONE HAVING LARGEST v(j)
S307
OUTPUT ACTIVE SPACE INFORMATION INCLUDING LIST OF IMPORTANCE OF L ORBITALS j
END

FIG. 7

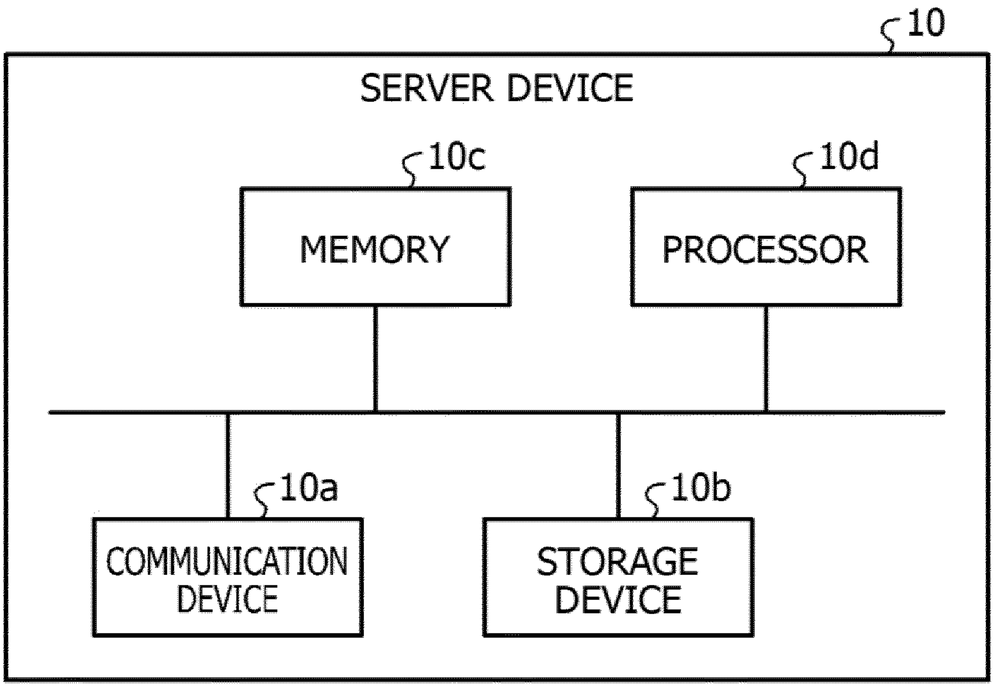
10
SERVER DEVICE
10c
MEMORY
10d
PROCESSOR
10a
COMMUNICATION DEVICE
10b
STORAGE DEVICE

Europäisches Patentamt
European Patent Office
Office européen des brevets

# EUROPEAN SEARCH REPORT

Application Number
EP 25 15 6982

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/013865 A1 (TAKAHASHI NORIHIKO [JP]) 11 January 2024 (2024-01-11)<br>* paragraphs [0007], [35ff.]; claim 1 *<br>----- | 1-15 | INV.<br>G16C10/00 |
| X | TANVI P GUJARATI ET AL: "Quantum Computation of Reactions on Surfaces Using Local Embedding",<br>ARXIV.ORG, CORNELL UNIVERSITY LIBRARY, 201 OLIN LIBRARY CORNELL UNIVERSITY ITHACA, NY 14853,<br>23 October 2023 (2023-10-23), XP091642039,<br>DOI: 10.1038/S41534-023-00753-1<br>* the whole document *<br>----- | 1-15 | |
| X | JULIA WESTERMAYR ET AL: "Machine learning for electronically excited states of molecules",<br>ARXIV.ORG, CORNELL UNIVERSITY LIBRARY, 201 OLIN LIBRARY CORNELL UNIVERSITY ITHACA, NY 14853,<br>10 July 2020 (2020-07-10), XP081892214,<br>DOI: 10.1021/ACS.CHEMREV.0C00749<br>* the whole document *<br>----- | 1-15 | TECHNICAL FIELDS SEARCHED (IPC)<br>G16C |

The present search report has been drawn up for all claims

2

| Place of search | Date of completion of the search | Examiner |
|---|---|---|
| Munich | 4 July 2025 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 15 6982

This annex lists the patent family members relating to the patent documents cited in the above-mentioned European search report. The members are as contained in the European Patent Office EDP file on
The European Patent Office is in no way liable for these particulars which are merely given for the purpose of information.

04-07-2025

| Patent document cited in search report | Publication date | Patent family member(s) | Publication date |
|---|---|---|---|
| US 2024013865 A1 | 11-01-2024 | CN 116490928 A | 25-07-2023 |
| | | EP 4243025 A1 | 13-09-2023 |
| | | JP 7506330 B2 | 26-06-2024 |
| | | JP WO2022097298 A1 | 12-05-2022 |
| | | US 2024013865 A1 | 11-01-2024 |
| | | WO 2022097298 A1 | 12-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022097298 A
- JP 2012032908 A
- US 20200349459
- US 20160378955